# EUROPEAN PATENT APPLICATION

(11) **EP 3 586 762 A1**
(43) Date of publication of application: **01.01.2020**
(21) Application number: 18180345.3
(22) Date of filing: 28.06.2018
(51) Int. Cl.: A61B 17/00, A61B 17/12, A61M 25/00

(54) **CANNULA FOR MINIMALLY INVASIVE SURGICAL TRICUSPID VALVE REPAIR**

(71) Applicant: Medinice S.A., 25-663 Kielce (PL)
(72) Inventor: Suwalski, Piotr, 02-593 Warszawa (PL)
(74) Representative: Bartula-Toch, Marta

(57) **Abstract**

A cannula for minimally invasive surgery, comprising two coaxial tubes - an outer short one (2) and an inner long one (3), of which the inner long tube (3) is slidably movable relative to the outer short tube (2), and a head (4) which in the folded position has a cone shape with an axial inlet hole (5) in the axis of the upper base, while in the unfolded position, with the outer short tube (2) slid off in the distal direction, the head (4) has the shape of a cup extending outwardly. The outer edge of the cup has a diameter about three times the inner diameter of the cannula (1). The outer short tube (2) is equipped with at least one rigid tie rod (8) the free end of which extends along the inner wall of the inner long tube (3) beyond its distal end.

## Description

The object of the invention is a cannula for minimally invasive surgical tricuspid valve repair.

Cannulation is a procedure for placing a cannula, i.e. a special tube in the arterial vessel, most often in the aorta or the femoral artery, and in the venous vessel for receiving and feeding blood from a patient to the apparatus providing extracorporeal circulation. For the surgeries on tricuspid valve, it is necessary to selectively cannulate both the superior vena cava (SVC) and the inferior vena cava (IVC) so that blood does not flow through the right atrium, which allows opening thereof and reaching the tricuspid valve undergoing surgery. In the standard procedure of tricuspid valve repair, blood flow to and from the SVC and the IVC is cut off before the blood reaches the right atrium. This procedure is necessary to isolate the right atrium with the tricuspid valve, and at the same time to protect other organs and tissues from death. Method of isolation, i.e. closing, clamping of the main veins around the cannula so that the blood flows only inside the cannula and not through the lumen of the vessel is essential for the cannulation. Clamping from outside by means of so-called snuggers is typical for standard surgeries i.e. by sternotomy. However, it can be very difficult or even impossible in the case of minimally invasive surgeries consisting of accessing the heart and valve by an incision having a few centimetres in the side wall of the chest without affecting the bones and of replacing the valve under the control of an endoscopic camera using special long tools. Such surgeries are associated with a different method of connecting the extracorporeal circulation, with another place of cannulation (vein and femoral artery in the groin) and, therefore, with the need to use completely different cannulas.

On the market, there are venous cannulas for minimally invasive surgery. Adding a balloon or a special collar to the cannula in its distal part, which allows the main vein to be closed somewhat from the inside, gives the same effect as clamping the vein from the outside and, therefore, allows minimally invasive surgery.

Normally, two cannulas are used for minimally invasive tricuspid valve surgery, one for the inferior vena cava, which is inserted into the femoral artery, and the other for the superior vena cava, which is inserted through the jugular vein. However, attempts have been made to develop a cannula design which would drain blood from both veins at the same time.

So, from patent No. US 6,086,557, a venous cannula is known which is intended to drain the patient's blood during cardiac surgery and has two branches at the end of the main part of the cannula. Around the middle of each branch, there is an occlusive balloon which, after filling, seals the blood vessel from the inside and, thereby, prevents blood flow along the cannula on its outer side.

From US patent No. US 5,800,375, a cannula and a method of cannulation of blood vessels during heart surgery are known. The cannula is equipped with two occlusive balloons sealing the main vein from the inside.

From patent application No. CN102475556, a cannula for the main vein is known which has an umbrella-shaped ring made of a soft silicone.

The essence of the invention consists in that the cannula is formed by two coaxial tubes - an outer short one and an inner long one, of which the inner long tube is slidably movable relative to the outer short tube, and its proximal end is constituted by a head. The head in the folded position has a cone shape with an axial inlet hole in the axis of the upper base, while in the unfolded position, with the outer short tube slid off in the distal direction, the head has the shape of a cup extending outwardly the outer edge of which has a diameter about three times the inner diameter of the cannula. The outer short tube is equipped with at least one rigid tie rod the free end of which extends along the inner wall of the inner long tube beyond its distal end.

Preferably, the cannula head is made of a non-reactive, resilient and stretchable material.

Preferably, the cannula head is made of silicone.

Preferably, the diameter of an inlet hole of the head constitutes from 50 to 75% of the outer diameter of the cannula.

Preferably, the head is equipped, from the inside, with an openwork resilient frame attached to the upper edge of the head.

Preferably, the head frame is made of a thin, resilient material resistant to external conditions.

Preferably, the frame components are curved in the shape of triangles the vertices of which are freely disposed inside the head.

Preferably, the inner long tube has, below the head, at least one longitudinal through hole.

Preferably, on a central section of the inner long tube, having a length of 10 to 20%, there are transverse holes in the walls, evenly spaced around its circumference.

The greatest advantage of the solution according to the invention is the fact that it allows, under conditions of minimally invasive cardiac surgery, selective draining of blood from the inferior and superior vena cava, which is necessary for tricuspid valve surgery. The cannula according to the invention is introduced only through the femoral vein, without the need for cannulation of other vessels (for example the jugular vein), and also does not require sealing from the outside of the cannula in the superior vena cava, which is a time-consuming procedure, and in certain anatomical and clinical conditions, for example in the case of reoperation surgery, is risky and impossible to conduct.

The cannula application is relatively simple and undoubtedly reduces the surgery time, which is of great importance for the condition of the cardiac patient and for the speed of his recovery after procedure. Head design allows for efficient passage through the veins, and at the same time it allows for tight cutting off of the blood flow to the right atrium. Variable outer diameter of the head gives the possibility to adjust its size to the inner diameter of the vein.

The simple design of the cannula according to the invention will also be important for the cost of its manufacture, and therefore for the final price.

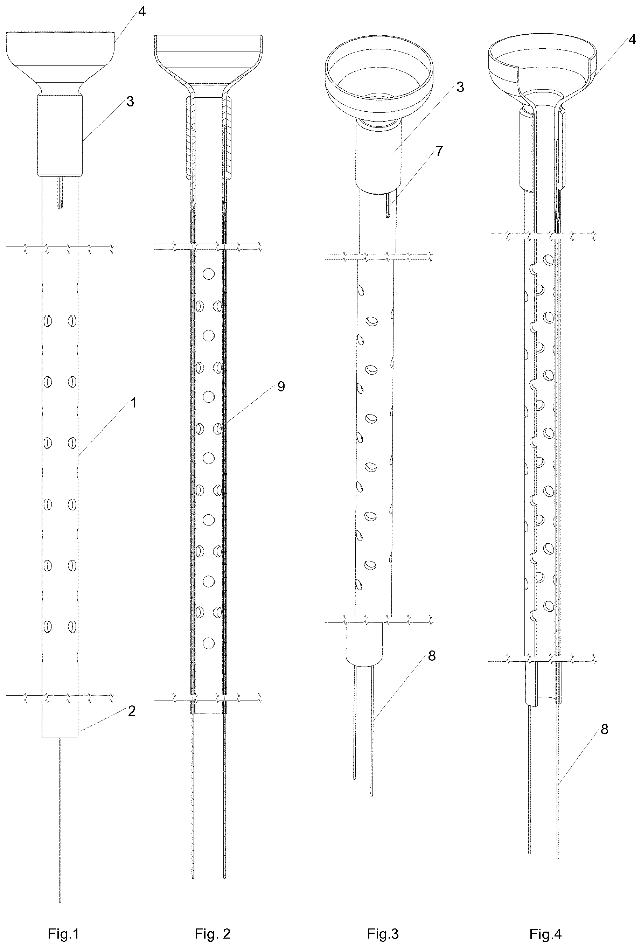

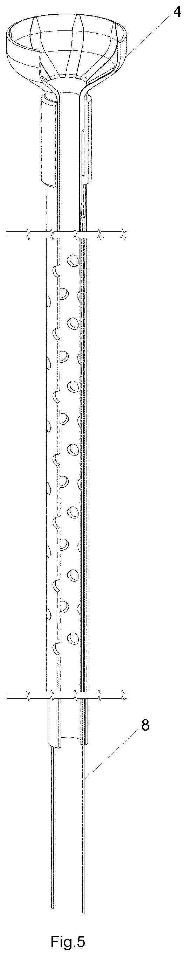

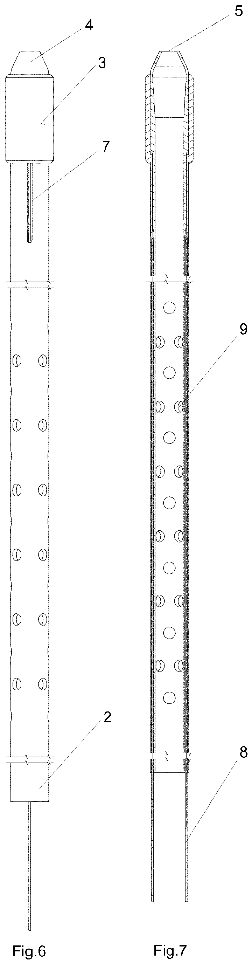

The object of the invention is presented in an embodiment in the drawing, where Fig. 1 is a view of the cannula with its head opened in a side view, Fig. 2 - a vertical section of the cannula with its head opened, Fig. 3 - an axonometric view of the cannula with its head opened, Fig. 4 - a half-view-half-section of the cannula with its head opened, Fig. 5 - a half-view-half-section of the cannula with its head opened and the frame, Fig. 6 - a side view of the cannula with its head closed, Fig. 7 - a cross-section of the cannula with its head closed and the frame.

### Example I

The cannula is formed by two coaxial tubes - an outer short one 2 and an inner long one 3, of which the inner long tube 3 is slidably movable relative to the outer short tube 2. The proximal end of the cannula 1 is constituted by a head 4 which in the folded position has a cone shape with an axial hole 5 in the axis of the upper base. The diameter of the hole 5 is proportional to the inner diameter of the cannula and constitutes 60% of the outer diameter of the cannula 1, thanks to which it ensures free blood flow into the cannula 1. In the unfolded position, with the outer short tube 2 slid off in the distal direction, the head 4 has the shape of a cup extending outwardly the outer edge of which has a diameter three times the inner diameter of the cannula. The head 4 of the cannula 1 is made of a resilient and stretchable material. On a central section of the inner long tube 3, having a length of 10 to 20%, there are transverse holes 9 in the walls, evenly spaced around its circumference. The inner short tube 2 has two rigid tie rods 8 the free ends of which equipped with ergonomic handles through longitudinal through holes 7 are introduced to the interior of the cannula 1, and then are guided along walls beyond it distal end.

The cannula 1 with its head 4 closed is introduced into the femoral vein, then, having reached the heart, the right vestibule is passed, and the head 4 of the cannula is placed in the superior vena cava. Then, by pulling the tie rods 8, the outer short tube 2 is pulled down below the head 4 in order to reveal it in its entirety. Then, flexible and resilient walls of the head 4 expand and develop into a cup the upper wall parts of which adhere to the inner walls of the vein, thereby preventing the flow of blood along the outer walls of the cannula 1. A connector, located at the distal end of the cannula, is connected to the apparatus providing extracorporeal circulation. After surgery, the outer short tube 2 is slid back towards the head 4, thereby leading to its closure, then the cannula 1 is removed from the vein.

### Example II

The cannula is formed by two coaxial tubes - an outer short one 2 and an inner long one 3, of which the inner long tube 3 is slidably movable relative to the outer short tube 2. The proximal end of the cannula 1 is constituted by a head 4 which in the folded position has a cone shape with an axial hole 5 in the axis of the upper base. The diameter of the hole 5 is proportional to the inner diameter of the cannula and constitutes 65% of the outer diameter of the cannula 1, thanks to which it ensures free blood flow into the cannula 1. In the unfolded position, with the outer short tube 2 slid off in the distal direction, the head 4 has the shape of a cup extending outwardly the outer edge of which has a diameter three times the inner diameter of the cannula 1. In addition, the head 4 is equipped, from the inside, with an openwork resilient frame 6 attached to the upper edge of the head 4, made of a thin, resilient wire or plastic bent in the shape of triangles the vertices of which are freely disposed inside the inner long tube 3. The head 4 of the cannula 1 is made of a resilient and stretchable material. On a central section of the inner long tube 3, having a length of 10 to 20%, there are transverse holes 9 in the walls, evenly spaced around its circumference. The inner short tube 2 has two rigid tie rods 8 the free ends of which equipped with ergonomic handles through longitudinal through holes 7 are introduced to the interior of the cannula 1, and then are guided along walls beyond it distal end.

The cannula 1 with its head 4 closed is introduced into the femoral vein, then, having reached the heart, the right vestibule is passed, and the head 4 of the cannula is placed in the superior vena cava. Then, by pulling the tie rods 8, the outer short tube 2 is pulled down below the head 4 in order to reveal it in its entirety. Then, the frame 6 expands and stretches walls of the head 4 which develops into a cup the upper wall parts of which adhere to the inner walls of the vein, thereby preventing the flow of blood along the outer walls of the cannula 1. A connector, located at the distal end of the cannula, is connected to the apparatus providing extracorporeal circulation. After surgery, the outer short tube 2 is slid back towards the head 4, thereby leading to its closure, then the cannula 1 is removed from the vein.

## Claims

1. A cannula for minimally invasive surgical tricuspid valve repair, which is constituted by at least one flexible tube equipped with a connector, **characterised in that** the cannula (1) is formed by two coaxial tubes - an outer short one (2) and an inner long one (3), of which the inner long tube (3) is slidably movable relative to the outer short tube (2), and its proximal end is constituted by a head (4) which in the folded position has a cone shape with an axial inlet hole (5) in the axis of the upper base, while in the unfolded position, with the outer short tube (2) slid off in the distal direction, the head (4) has the shape of a cup extending outwardly the outer edge of which has a diameter about three times the inner diameter of the cannula (1), and the outer short tube (2) is equipped with at least one rigid tie rod (8) the free end of which extends along the inner wall of the inner long tube (3) beyond its distal end.

2. The cannula according to claim 1, **characterised in that** the head (4) of the cannula (1) is made of a resilient and stretchable material.

3. The cannula according to claim 2, **characterised in that** the head (4) of the cannula (1) is made of silicone.

4. The cannula according to claim 1, **characterised in that** the diameter of the hole (5) constitutes from 50 to 75% of the outer diameter of the cannula (1).

5. The cannula according to claim 1, **characterised in that** the head (4) is equipped, from the inside, with an openwork resilient frame (6) attached to the upper edge of the head (4).

6. The cannula according to claim 5, **characterised in that** the frame (6) of the cannula (4) is made of a thin, resilient metal wire or plastic resistant to external conditions.

7. The cannula according to claim 5, **characterised in that** components of the frame (6) are bent in the shape of triangles the vertices of which are freely disposed inside the inner long tube (3).

8. The cannula according to claim 1, **characterised in that** the inner long tube (3) has, below the head (4), at least one longitudinal through hole (7).

9. The cannula according to claim 1, **characterised in that** on a central section having a length of 10 to 20% of the total length of the inner long tube (3), there are transverse holes (9) in the walls, evenly spaced around its circumference.

## Amended claims

### Amended claims in accordance with Rule 137(2) EPC.

1. A cannula for minimally invasive surgical tricuspid valve repair, which is constituted by at least one flexible tube equipped with a connector, **characterised in that** the cannula (1) is formed by two coaxial tubes - an outer short one (2) and an inner long one (3), of which the inner long tube (3) is slidably movable relative to the outer short tube (2), and its distal end is constituted by a head (4) which in the folded position has a cone shape with an axial inlet hole (5) in the axis of the upper base, while in the unfolded position, with the outer short tube (2) slid off in the proximal direction, the head (4) has the shape of a cup extending outwardly the outer edge of which has a diameter about three times the inner diameter of the cannula (1), and the outer short tube (2) is equipped with at least one rigid tie rod (8) the free end of which extends along the inner wall of the inner long tube (3) beyond its proximal end.

2. The cannula according to claim 1, **characterised in that** the head (4) of the cannula (1) is made of a resilient and stretchable material.

3. The cannula according to claim 2, **characterised in that** the head (4) of the cannula (1) is made of silicone.

4. The cannula according to claim 1, **characterised in that** the diameter of the hole (5) constitutes from 50 to 75% of the outer diameter of the cannula (1).

5. The cannula according to claim 1, **characterised in that** the head (4) is equipped, from the inside, with an openwork resilient frame (6) attached to the upper edge of the head (4).

6. The cannula according to claim 5, **characterised in that** the frame (6) of the cannula (4) is made of a thin, resilient metal wire or plastic resistant to external conditions.

7. The cannula according to claim 5, **characterised in that** components of the frame (6) are bent in the shape of triangles the vertices of which are freely disposed inside the inner long tube (3).

8. The cannula according to claim 1, **characterised in that** the inner long tube (3) has, below the head (4), at least one longitudinal through hole (7).

9. The cannula according to claim 1, **characterised in that** on a central section having a length of 10 to 20% of the total length of the inner long tube (3), there are transverse holes (9) in the walls, evenly spaced around its circumference.
